# EUROPEAN PATENT APPLICATION

(11) **EP 3 109 634 A1**
(43) Date of publication of application: **28.12.2016**
(21) Application number: 15305966.2
(22) Date of filing: 23.06.2015
(51) Int. Cl.: G01N 33/00, G01N 21/35, G01N 21/3504

(54) **AN AUTOMATED CALIBRATION OR MEASUREMENT DEVICE ADAPTED FOR THE DETERMINATION OF WATER ISOTOPIC COMPOSITION OF AN UNKNOWN LIQUID AND VAPOR SAMPLE, ASSOCIATED AUTOMATED MEASUREMENT PROCESS**

(71) Applicant: COMMISSARIAT A L'ENERGIE ATOMIQUE ET AUX ENERGIES ALTERNATIVES, 75015 Paris (FR)
(72) Inventor: Steen-Larsen, Hans-Christian, 75013 Paris (FR)
(74) Representative: Cabinet Camus Lebkiri

(57) **Abstract**

The invention relates to an automated device adapted for the determination of the isotopic composition of an unknown liquid sample, comprising:
- at least a first branch (BR₁) comprising :
o a first vaporizer (VP₁),
o a controlled valve system (VS₁),
o a first container (CT₁),
o a first circuit (C1₁),
o a first controlled valve (V1₁),

- first regulation means (MR1),
- second regulation means (MR2),
- a second circuit (C2),
- a third circuit (C3),
- a fourth circuit (C4).

## Description

### FIELD OF THE INVENTION

The present invention relates to an automated calibration or measurement device for the determination of water isotopic composition of an unknown liquid or vapor sample. More specifically, the present invention can be used to measure isotopic composition of an ambient air sample. The invention also concerns an automated measurement process of water isotopic composition of an unknown liquid or vapor sample.

### BACKGROUND

Many different devices for the determination of isotopic composition of an unknown sample have been designed.

More particularly, the article named "Calibrated high-precision ¹⁷O-excess measurements using cavity ring-down spectroscopy with laser-current-tuned cavity resonance" (Steig et al.) discloses a device used for the determination of water isotopic concentration in water samples.

The device disclosed in said article comprises a gas analyzer, an input of dry air which is connected to two pressure regulators. The first pressure regulator is connected to one of a plurality of vials containing samples to be analyzed using an autosampler. When a plurality of samples contained in vials must be analyzed, the uses of an autosampler does not permit rapids measurements. The second pressure regulator is connected to a vaporizer. The first pressure regulator connected to the dry air is utilized to push out a small amount of sample contained is said vials through a capillary which is connected to a vaporizer. The vaporizer is used to vaporize the small amount of sample punctured. The second pressure regulator connected to the dry air is used to mix said dry air with the vaporized sample in the vaporizer and thus to control the vapor concentration of the vaporized sample before its entry into the gas analyzer. Thus, the vaporized sample is transported to the gas analyzer to measure the water isotopic composition of the sample.

However, such device does not permit a rapid, controlled and continuous measurement of isotopic composition of an unknown sample nor measurements of several unknown samples.

### SUMMARY

An aspect of the invention is directed to a device that overcome that aforementioned drawbacks. Accordingly, an embodiment of the invention is directed to an automated device for a quick, a continuous and a precise determination of isotopic composition of an unknown sample.

To achieve this, a first aspect of the present invention is directed to an automated device adapted for the determination of the isotopic composition of an unknown liquid sample, comprising:
- at least a first branch comprising :
   o a first vaporizer comprising :
      ▪ a first input constructed and arranged for receiving a liquid sample to be vaporized ;
      ▪ a second input constructed and arranged for receiving a first gas flow such as air flow ;
      ▪ an output constructed and arranged for outputting the vaporized liquid sample pushed out by said first gas flow ;
   o a controlled valve system comprising an input connected to the output of the vaporizer, a first output adapted to be connected to a gas analyzer and a second output left in the open air, said controlled valve system being constructed and arranged to allow or block the passage of the vaporized liquid sample to the gas analyzer and to reach a stable humidity of the vaporized liquid sample before being transported to the gas analyzer;
   o a first container constructed and arranged for containing said liquid sample comprising :
      ▪ an input constructed and arranged for receiving a second gas flow such as air flow ;
      ▪ an output constructed and arranged for transporting the liquid sample from the first container;
   o a first circuit connecting the output of the first container to the input of the first vaporizer, said first circuit being constructed and arranged to bring the liquid sample to the first vaporizer;
   o a first controlled valve comprising an input connected to the input of the first container and an output left in the open air, said first controlled valve being constructed and arranged to allow or block the evacuating of the air contained in the first container by the output of the first controlled valve ;
- first regulation means of the first gas flow, said first regulation means being connected to the second input of the first vaporizer;
- second regulation means of the second gas flow, said second regulation means being connected to the input of the first container;
- a second circuit connected to the output of the controlled valve system ;
- a third circuit connecting the first regulation means to the second input of the first vaporizer;
- a fourth circuit connecting the second regulation means to the input of the first container.

As the device of the invention comprises controlled valves, said device can be controlled and permits a rapid and continuous measurement of isotopic composition of an unknown sample. More particularly, because of the controlled valve system, the vaporized sample which will be analyzed by the gas analyzer has the appropriate humidity concentration at its entry into said gas analyzer. Thus, it allows a rapid and precise measurement since the first amount of vaporized sample is introduced into the gas analyzer. Moreover, the device can be used as a calibration device or as a measurement device.

Advantageously, when the device comprises a plurality of branches mounted in parallel and separated by valves, the device allows a rapid measurement of isotopic composition of samples contained in each branch without using an autosampler.

Otherwise, for a measurement of isotopic composition of an unknown sample, two branches can be used for the calibration, each branch containing a liquid standard sample having a known isotopic composition. The measurement obtained is thus both more precise and accurate.

The device according to the first aspect of the invention may also have one or more of the characteristics below, considered individually or according to all of the technically possible combination:
The automated device comprises:
   - a gas analyzer comprising :
      o an input constructed and arranged for receiving a gas to be analyzed ;
      o an output constructed and arranged for evacuating said gas after analysis.
   - The automated device comprises an exhaust connected to the second circuit, said exhaust being constructed and arranged to evacuate the flow surplus of vaporized liquid sample in the second circuit before its entry into the input of the gas analyzer.
   - The controlled valve system of the first branch is a three-way valve.
   - The controlled valve system of the first branch is a combination of two two-way valves, said two two-way valves being constructed and arranged to be connected through a common point forming the input of the controlled valve system.
   - The first regulation means comprise:
      - first means constructed and arranged for delivering dry gas ;
      - a mass flow controller connected to the first means, said mass flow controller being constructed and arranged to control the mass flow of the dry gas from the first means, forming the first gas flow.
   - The first means constructed and arranged for delivering dry gas are either a first receptacle containing dry gas or a pump delivering gas connecting through a drying unit wherein said gas becomes dry.
   - The second regulation means comprise :
      - second means constructed and arranged for delivering compressed gas ;
      - a pressure regulator connected to the second means constructed and arranged for delivering compressed gas, said pressure regulator being constructed and arranged to regulate the pressure of the compressed gas from said second means constructed and arranged for delivering compressed gas, forming the second gas flow.
   - The second means constructed and arranged for delivering compressed gas are either a second receptacle containing compressed gas or a pump delivering compressed gas.
   - The automated device comprises a plurality of branches mounted parallel, each branch being connected, at one end, to the second circuit and, at the other end, to the fourth circuit and comprising :
      - a second controlled valve comprising an input and an output connected to the fourth circuit, said second controlled valve being constructed and arranged to allow or block the passage of the second gas flow to a first container;
      - a third controlled valve comprising an input connected to the third circuit and an output, said third controlled valve being constructed and arranged to allow or block the passage of the first gas flow to an input of a first vaporizer.
   - The first circuit is a capillary tube.
   - The automated device, comprising the gas analyzer, comprises a fourth controlled valve comprising :
      - a first input connected to a first output of a controlled valve system, said first input being constructed and arranged for receiving the vaporized liquid sample ;
      - a second input constructed and arranged for receiving ambient air;
      - an output connected to the first input of the gas analyzer, said output being constructed and arranged to allow the passage of the vaporized liquid sample to the gas analyzer or to allow the passage of ambient air from the second input to the gas analyzer.

A second aspect of the present invention is directed to a measurement process of isotopic composition of an unknown liquid sample using the automated device according to the first aspect of the invention, said measurement process comprising:
- a calibration step comprising the following steps :
   o introduction of the second gas flow, by the second regulation means, in the fourth circuit;
   o introduction of the first gas flow, by the first regulation means, in the third circuit;
   o connection of one of the inputs to one of the first outputs of one of the controlled valve systems by controlling said controlled valve systems, said connection allowing the passage of a liquid standard sample, from one of the containers, vaporized, by one of the vaporizers, in the second circuit;
   o connection of the first input to the output of the fourth controlled valve by controlling said fourth controlled valve (V4), said connection allowing the passage of the vaporized liquid standard sample to the analyzer;
   o measurement of isotopic composition of the vaporized liquid standard sample by the analyzer;
- a measurement step comprising the following steps :
   o connection of the second input to the output of the fourth controlled valve by controlling said fourth controlled valve, said connection allowing the passage of ambient air to the analyzer ;
   o measurement of isotopic composition in ambient air by the analyzer.

The device according to the second aspect of the invention may also have the characteristics below:
- The calibration step comprises the following steps :
   o connection of one of the inputs to one of the outputs of one of the third controlled valves said connection allowing the passage of the first gas flow from the third circuit to one of the vaporizers ;
   o connection of one of the inputs to one of the outputs of one of the second controlled valves, said connection allowing the passage of the second gas flow from the fourth circuit to one of the containers) and the passage of the liquid standard sample to one of the vaporizers through one of the first circuits.

### BRIEF DESCRIPTION OF THE DRAWINGS

The accompanying drawings are included to provide a further understanding of the invention and are incorporated in a constitute part of this specification, to illustrate embodiments of the invention and, together with the description, to explain the principles of the invention:
- Figure 1 represents an example of an automated device adapted for the determination of isotopic composition of an unknown sample according to an embodiment of the invention ;
- Figure 2 represents an example of an automated device adapted for the determination of isotopic composition of an unknown sample according to another embodiment of the invention than the figure 1 ;
- Figure 3 represents an example of an automated device adapted for the determination of isotopic composition of an unknown sample according to another embodiment than the figures 1 and 2 ;
- Figure 4 represents an example of a measurement step of a measurement process of isotopic composition of an ambient air sample using the automated device represented in the figure 3 according to an embodiment of the invention ;
- Figure 5 represents an example of sub-steps of the measurement step of the measurement process of the figure 4 ;
- Figure 6 represents an example of a calibration step of the measurement process of isotopic composition of an unknown sample using the automated device represented in the figure 3 according to an embodiment of the invention;
- Figures 7 to 10 represent examples of sub-steps of the calibration step of the figure 6.

### DESCRIPTION OF SPECIFIC EMBODIMENTS

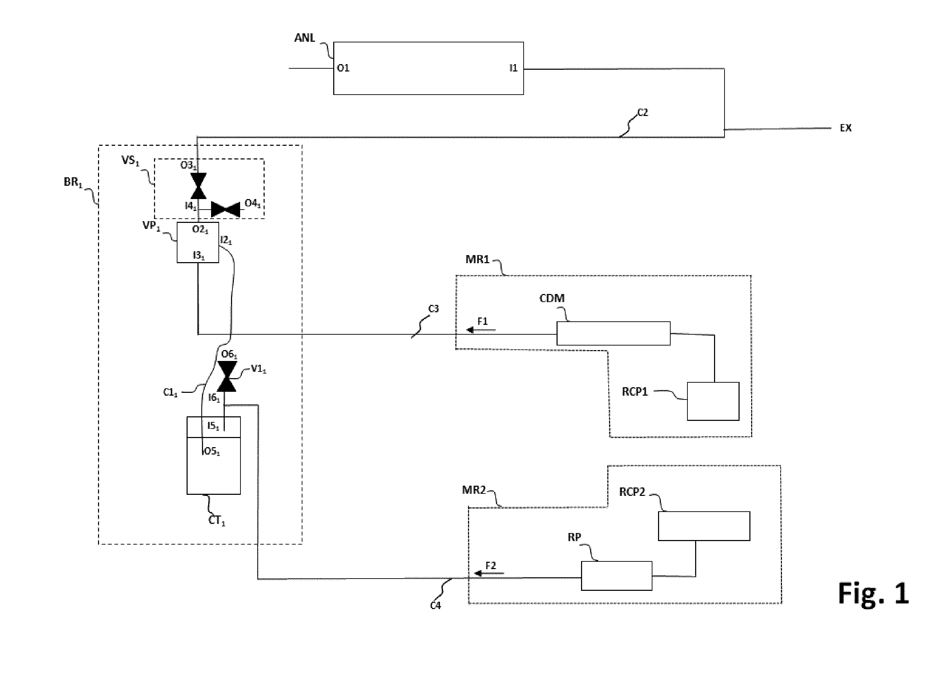

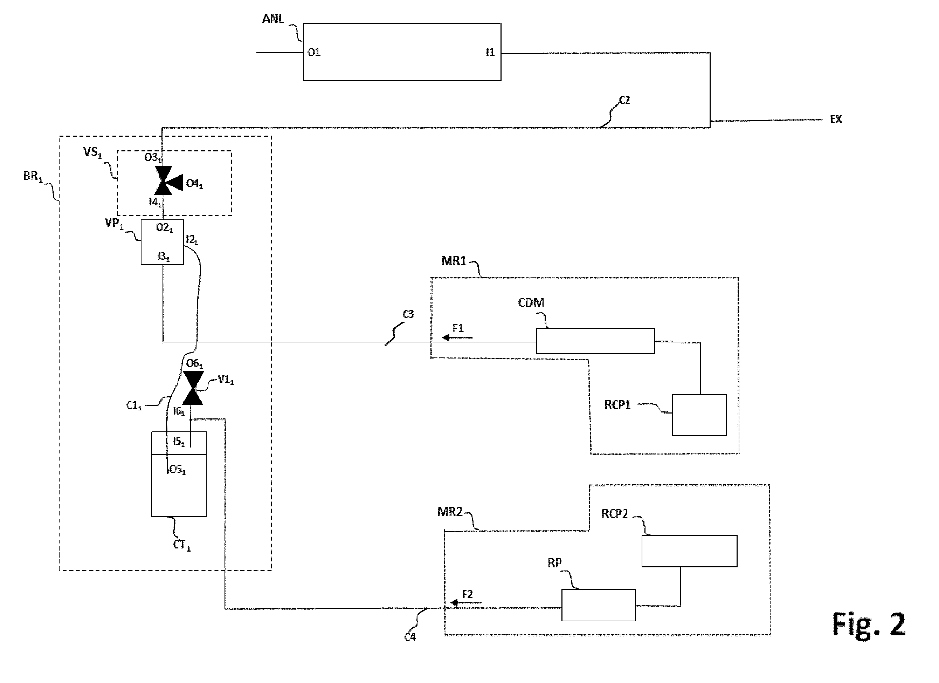

In reference to figures 1 and 2, examples of an automated device adapted for the determination of isotopic composition of an unknown sample according to two embodiments of the invention are represented. In a non-limiting embodiment, these devices comprise one first branch BR₁.

The automated devices represented in the figure 1 and in the figure 2 comprises:
- a gas analyzer ANL,
- the first branch BR₁ comprising:
   - a first container CT₁,
   - a first vaporizer VP₁,
   - a first circuit C1₁,
   - a controlled valve system VS₁,
   - a first controlled valve V1₁,
- circuits (C2, C3, C4),
- regulation means (MR1, MR2),
- an exhaust EX.

We remind that, in the embodiments of the invention represented in the figures 1 and 2, the automated device can be used as a calibration device or as a measurement device. Indeed, when the device is used as a calibration device, the first branch BR₁ contains a liquid standard sample whereas when the device is used as a measurement device, the first branch BR₁ contains an unknown liquid.

A "gas analyzer" means a device capable of measuring an isotopic composition of a gas. In a non-limiting embodiment, the gas analyzer ANL is a spectroscopy analyzer.

The gas analyzer ANL comprises an input I1 that receives a gas to be analyzed and an output 01 that evacuates said gas after its analysis. In a non-limiting embodiment of the invention, the gas to be analyzed is a gas loaded with water vapor. In the embodiment represented in the figures 1 and 2, the gas analyzer ANL is used to measure isotopic composition of a liquid standard sample when the device is used as a calibration device or of an unknown liquid sample when the device is used as a measurement device.

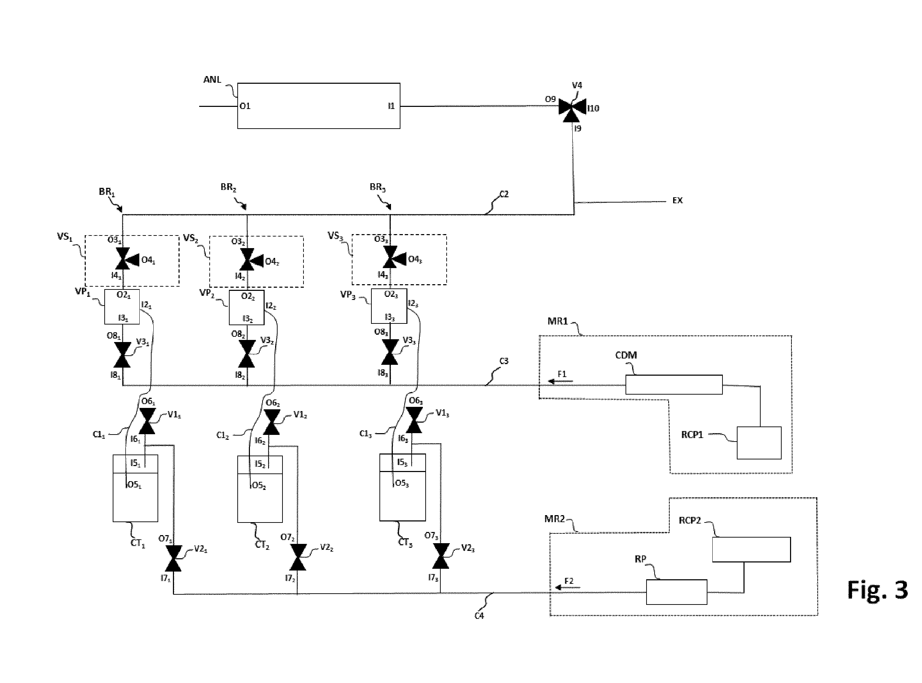

A "branch" means a part of a device that contains a plurality of elements. In the figure 1, said elements are the first container CT₁, the first vaporizer VP₁, the first circuit C1₁ the controlled valve system VS₁ and the first controlled valve V1₁. In a non-limiting embodiment, the automated device comprises one branch BR₁. In another non-limiting embodiment, the automated calibration device comprises a plurality of branches (BR₁, BR₂, BR₃) mounted parallel as represented in the figures 3 to 10.

A «container» means a canister or a vial which can be closed so that said container is air and leak tight. Besides, a container has one or a plurality of inputs and outputs. Furthermore, a container is made of a material that does not react, for a short or a long period, with a sample contained therein. For example, said material is glass, metal, plastic, teflon....

The first container CT₁ contains, in a first embodiment, a liquid standard sample during a calibration. In a second embodiment, the first container CT₁ contains an unknown liquid sample during a measurement. The first container CT₁ comprises an input I5₁ that receives a second gas flow F2. Moreover, the first container CT₁ comprises an output O5₁ that transports a liquid standard sample, during a calibration, or an unknown liquid sample, during a measurement, from the first container CT₁.

A «vaporizer» means a device which transforms a liquid into vapor/gas by applying the vaporization temperature of said liquid. For example, for a water sample, the vaporization temperature is about more than 100 degree Celsius. In a non-limiting embodiment, the first vaporizer VP₁ is used to vaporize water liquid into water vapor. In a non-limiting embodiment, the first vaporizer VP₁ is a VICI® (Valco Instruments Co. Inc.) tee-piece made with stainless steel (product number ZT1M). The VICI® ZT1 M is particularly interesting because it is an off-the shelf piece which has a very small inner dead-volume. In a non-limiting embodiment, the said first vaporizer VP₁ has a diameter of 0.50 mm (millimeter). In a non-limiting embodiment, said first vaporizer VP₁ a length of 1/16 inch (1 inch= 2.54 centimeter). In a non-limiting embodiment, the first vaporizer is heated to 170 degree Celsius.

The first vaporizer VP₁ of the first branch BR₁ comprises a first input I2₁ that receives, during a calibration, a liquid standard sample to be vaporized. During a measurement, said first input I2₁ of the first vaporizer VP₁ of the first branch BR₁ receives an unknown liquid sample to be vaporized. Furthermore, said first vaporizer VP₁ comprises a second input I3₁ that receives a first gas flow F1. Finally, the first vaporizer VP₁ of the first branch BR₁ includes an output O2₁ that evacuates, during a calibration, the vaporized standard sample. During a measurement, said output O2₁ of the first vaporizer VP₁ of the first branch BR₁ evacuates the vaporized unknown sample.

"Regulation means" of a gas flow means a device that control the mass flow of the gas by applying a fixed mass flow or a fixed pressure into said gas. As represented in the figures 1 to 10, the automated device comprises first regulation means MR1 and second regulation means MR2.

The first regulation means MR1 comprises a first receptacle RCP1 that contains dry gas such as dry air, dry nitrous, dry neon, dry helium or dry argon. In a non-limiting embodiment, the first receptacle RCP1 is a pump delivering gas connecting through a drying unit wherein said gas becomes dry. The first regulation means MR1 also contains a mass flow controller CDM connected to the first receptacle RCP1. The mass flow controller CDM controls the mass flow of the dry gas from the first receptacle RCP1, forming the first gas flow F1. The mass flow controller CDM regulates the mass flow of the first gas flow F1 in a range between [0 L.min⁻¹; 10 L.min⁻¹]. More generally, the mass flow controller CDM regulates the mass flow of the first gas flow F1 in a range between 0 L.min⁻¹ and much more than 10 L.min⁻¹. Indeed, the mass flow of the first gas flow is chosen according to the humidity concentration of the sample desired at its entry in the analyzer ANL. By increasing the mass flow of the dry air, the sample is being diluted more and the humidity decreases whereas the decrease of the mass flow of the dry air allows an increase of the humidity. Moreover, the mass flow of the dry air has to be larger than the mass flow needed by the analyzer ANL.

The first regulation means MR1 of the first gas flow F1 is connected to the second input I3₁ of the first vaporizer VP₁. Indeed, the first gas flow F1 is used to transport, during a calibration, the vaporized standard sample from the first vaporizer VP₁ to the analyzer ANL with a fixed mass flow. During a measurement, the first gas flow F1 is used to transport the vaporized unknown sample from the first vaporizer VP₁ to the analyzer ANL with a fixed mass flow.

The second regulation means MR2 comprises a second receptacle RCP2 that contains, in a non-limiting embodiment, a compressed gas such as compressed air. In another non-limiting embodiment, the second receptacle RCP2 contains a dry gas such as dry air. In another non-limiting embodiment, the second receptacle RCP2 is a pump delivering compressed gas or a pump delivering gas connecting through a compressing unit wherein said gas becomes compressed. The second regulation means MR2 also contains a pressure regulator RP connected to the second receptacle RCP2. The pressure regulator RP regulates the pressure of the compressed gas or of the dry gas from said second receptacle RCP2, forming the second gas flow F2. In a non-limiting embodiment, the regulator pressure RP is controlled electronically. In another non-limiting embodiment, the regulator pressure RP is controlled manually. We note that using an electronically regulator pressure RP allows for a more dynamic autonomous variation of the generated humidity level.

The second regulation means MR2 of the second gas flow F2 is connected to the input I5₁ of the first container CT₁. Indeed, the second gas flow F2 is used to increase the pressure in the headspace of the first container CT₁. The increase of the pressure in said headspace of the first container CT₁ exerts a force on the surface of the liquid standard sample or of the unknown liquid sample that is then pushed through a first circuit C1₁ of the first branch BR₁ also named capillary C1₁. In a non-limiting embodiment, the pressure range used in the automated device is varying between 0 and 0.2 bar above ambient pressure. A higher pressure results in more liquid being pushed through the first circuit C1₁. More generally, the pressure ranged in the automated device is varying between 0 bar and much more than 2 bar, the pressure needed to push the sample through the first circuit C1₁ depends on the length and the inner diameter of said first circuit C1₁. It is noted that as soon as the appropriate pressure set by the pressure regulator RP is reached, the second gas flow F2 is stopped because the pressure in the fourth circuit C4 and in the first container CT₁ has been equalized and hence no pressure driven flow exists.

A "first circuit" also named «capillary» means a tube with a very small inside diameter that delivers a very small amount of liquid. In a non-limiting embodiment, the first circuit is a fused silica capillary. In a non-limiting embodiment, the first circuit C1₁ of the first branch BR₁ has an inner diameter between 50 and 150 micrometers. In a non-limiting embodiment, the first circuit C1₁ of the first branch BR₁ has a length of 1 meter. We remind that no exact dimensions of the first circuit C1₁ of the first branch BR₁ are needed as the pressure in the headspace of the first container CT₁ can be regulated to accommodate a wide variety of said first circuit C1₁.

The first circuit C1₁ of the first branch BR₁ also called capillary C1₁ of the first branch BR₁ brings the liquid standard sample, during a calibration, or the unknown liquid sample, during a measurement, to the first vaporizer VP₁. The first circuit C1₁ of the first branch BR₁ connects the output O5₁ of the first container CT₁ to the input I2 of the first vaporizer VP₁. The purpose of the capillary C1₁ of the first branch BR₁ is to deliver a very small amount of liquid standard sample or unknown liquid sample to the first vaporizer VP₁.

A "valve" means a device that controls the flow of a gas or of a liquid through a pipe. There are different types of valves such as three-way valves and two-way valves.

A "controlled valve" is a non-manual valve. In a non-limiting embodiment, said controlled valve is a solenoid valve that is an electromechanically operated valve, said solenoid valve being controlled by an electric current through a valve that allow an automation of the calibration or measurement device.

Thus, the first branch BR₁ comprises the controlled valve system VS₁ and the first controlled valve V1₁. In a non-limiting embodiment represented in the figure 1, the controlled valve system VS₁ is a combination of two two-way valves. In another non-limiting embodiment represented in the figure 2, the controlled valve system VS₁ is a three-way valve.

The controlled valve system VS₁ comprise an input I4₁ connected to the output O2₁ of the first vaporizer VP₁. Furthermore, said first controlled valve V1₁ comprise a first output O3₁ connected to the analyzer ANL and a second output O4₁ left in the open air. For the controlled valve system VS₁ represented in the figure 1, the input I4₁ of said controlled valve system VS₁ is connected to the said two two-way valves. The controlled valve system VS₁ allows or blocks the passage of the liquid standard sample or of the unknown liquid sample to the first vaporizer VP₁ and reach a stable humidity flow of the vaporized standard sample or of the vaporized unknown sample. Indeed, the connection of the input I4₁ to the second output O4₁ of the controlled valve system VS₁ stabilizes the humidity flow of the vaporized standard sample or of the vaporized unknown sample before its entry into the analyzer. We note that, in the embodiment represented in the figure 1, when the two two-way valves are closed, the first flow F1 is on and the input I6₁ and the output O6₁ of the first controlled valve V1₁ are open, an overpressure in the first vaporizer VP₁ is created which pushes back the liquid standard sample or the unknown liquid sample into the first circuit C1₁.

The first controlled valve V1₁ comprises an input I6₁ connected to the input I5₁ of the first container CT₁ and an output O6₁ left in the open air. The first controlled valve V1₁ allows or blocks the evacuating of the air contained in the first container CT₁ by the output O6₁ of the first controlled valve V1₁. Indeed, when the output O6₁ of the first controlled valve V1₁ is opened and the second flow F2 is stopped, the pressure in the headspace of the first container CT₁ fixed by the second regulation means MR2 decreases until the ambient air pressure is reached. This decrease of pressure leads to stop the pushing of the liquid standard sample or of the unknown liquid sample through the first circuit C1₁ of the first branch BR₁ and consequently the calibration step or the measurement step.

A "circuit" (C2, C3, C4) means a tube made with a material that has no interaction with the liquid standard sample or an unknown sample or ambient air which is going to be analyzed. In a non-limiting embodiment, said material is a stainless steel. In a non-limiting embodiment, the outer diameter of the circuit (C3, C4) is 1/8 inch. In another non-limiting embodiment, the outer diameter of the circuit (C3, C4) is between [1/16 inch, 1/4 inch].

The second circuit C2 connects the output O3₁ of the controlled valve system VS₁ to the input I1 of the analyzer ANL. The second circuit C2 brings the vaporized standard sample or the vaporized unknown sample to the analyzer ANL.

A third circuit C3 connects the first regulation means MR1 to the second input I3₁ of the first vaporizer VP₁.

A fourth circuit C4 connects the second regulation means MR2 to the input I5₁ of the first container CT₁.

The exhaust EX connects the second circuit C2 and allows the surplus of the vaporized standard sample or of the vaporized unknown sample to escape before the needed amount of flow enters the analyzer ANL.

In reference to figure 3, an example of an automated device for the determination of isotopic composition of an unknown liquid sample is represented.

The automated device represented in the figure 3 comprises:
- the gas analyzer ANL,
- a plurality of branches: the first branch BR₁, a second branch BR₂, a third branch BR₃, each branch (BR₁, BR₂, BR₃) comprising:
   - containers (CT₁, CT₂, CT₃),
   - vaporizers (VP₁, VP₂, VP₃),
   - first circuits (C1₁, C1₂, C1₃),
   - controlled valve systems (VS₁, VS₂, VS₃)
   - controlled valves (V1₁, V1₂, V1₃, V2₁, V2₂, V2₃, V3₁, V3₂, V3₃),
- circuits (C2, C3, C4),
- regulation means (MR1, MR2),
- fourth controlled valve V4,
- an exhaust EX.

In this case, the device can be used as a calibration device or as a measurement device. Indeed, when the device is used as a calibration device, at least one branch contains a liquid standard sample to be analyzed by the analyzer ANL. When the device is used as a measurement device, in one non-limiting embodiment of the invention, the device comprises a valve, in this case the fourth controlled valve V4, which allows an entry of an ambient air sample to be analyzed by the analyzer ANL. In another embodiment of the invention, at least one branch contains an unknown liquid to be analyzed by the analyzer ANL.

A "plurality of branches" means that the number of branches is between [1; n], n being higher than 1.

As represented in the figure 3, three branches (BR₁, BR₂, BR₃) are mounted parallel, each branch (BR₁, BR₂, BR₃) comprising the same elements arranged in the same way than the first branch BR₁ described above.

Thus, the first branch BR₁ comprises the first container CT₁, the first vaporizer VP₁, the first circuit C1₁, the controlled valve system VS₁ and the first controlled valve V1₁. The second branch BR₂ comprises a second container CT₂, a second vaporizer VP₂, a first circuit C1₂, a controlled valve system VS₂ and a first controlled valve V1₂.

The third branch BR₃ comprises a third container CT₃, a third vaporizer VP₃, a first circuit C1₃, a controlled valve system VS₃ and a first controlled valve V1₃.

The three branches (BR₁, BR₂, BR₃) are connected, at one end, to the second circuit C2 and, to the other end, to the fourth circuit C4. Moreover, each second input (I3₁, I3₂, I3₃) of each vaporizer (VP₁, VP₂, VP₃) of each branch (BR₁, BR₂, BR₃) is connected to the third circuit C3.

Furthermore, said three branches (BR₁, BR₂, BR₃) comprise a second controlled valve (V2₁, V2₂, V2₃) comprising an input (I7₁, I7₂, I7₃) connected to the fourth circuit C4 and an output (O7₁, O7₂, O7₃) connected to the related input (I6₁, I6₂, I6₃) of the first controlled valve (V1₁, V1₂, V1₃). The second controlled valve (V2₁, V2₂, V2₃) allows or blocks the passage of the second gas flow F2 to the container (CT₁, CT₂, CT₃). Moreover, the three branches (BR₁, BR₂, BR₃) comprise a third controlled valve (V3₁, V3₂, V3₃) comprising an input (I8₁, I8₂, I8₃) connected to the third circuit C3 and an output (O8₁, O8₂, O8₃) connected to the related input (I3₁, I3₂, I3₃) of the vaporizer (VP₁, VP₂, VP₃). The third controlled valve (V3₁, V3₂, V3₃) allows or blocks the passage of the first gas flow F1 to the vaporizer (VP₁, VP₂, VP₃).

Thus, the first branch BR₁ comprises the second controlled valve V2₁, which includes an input I7₁ and an output O7₁. The first branch BR₁ comprises the third controlled valve V3₁, which includes an input I8₁ and an output O8₁.

The second branch BR₂ comprises the second controlled valve V2₂, which includes an input I7₂ and an output O7₂. The second branch BR₂ comprises the third controlled valve V3₂, which includes an input I8₂ and an output O8₂.

The third branch BR₃ comprises the second controlled valve V2₃, which includes an input I7₃ and an output O7₃. The third branch BR₃ comprises the third controlled valve V3₃, which includes an input I8₃ and an output O8₃.

Each branch (BR₁, BR₂, BR₃) contains a liquid standard sample or an unknown liquid sample in each corresponding container (CT₁, CT₂, CT₂).

For example, the first container CT₁ of the first branch BR₁ contains a first liquid standard sample and the second container CT₂ of the second branch BR₂ contains a second liquid standard sample. The first and the second liquids standards samples have a different isotopic composition from each other. The third container CT₃ of the third branch BR₃ contains an unknown liquid sample. We note that, for a calibration step, the liquid standard samples which are chosen depend on the kind of unknown sample that will be analyzed. Thus, after a calibration step 100 in which each liquid standard sample is analyzed by the gas analyzer ANL, a measurement step 101 is proceeded in which the unknown liquid sample contained in the third container CT₃ is analyzed by the analyzer ANL. In a non-limiting embodiment, only one liquid standard sample contained in one container, for example CT₁, is analyzed during said calibration step 100. In another non-limiting embodiment, more than two liquid standard samples are analyzed during said calibration step 100, each liquid standard sample being contained in a container CTₙ of a branch BRₙ. In another non-limiting embodiment, the branches (BR₁, BR₂, BR₃) are only used to the calibration step 100, and so, the containers (CT₁, CT₂, CT₃) of the branches (BR₁, BR₂, BR₃) only contain liquid standard samples. In another non-limiting embodiment, , the branches (BR₁, BR₂, BR₃) are only used to the measurement step 101, and so, the containers (CT₁, CT₂, CT₃) of the branches (BR₁, BR₂, BR₃) only contain unknown liquid samples.

Otherwise, when a liquid standard sample or an unknown liquid sample is chosen to be analyzed, the branch which contains said liquid standard sample or said unknown liquid sample is activated by valves whereas the other branches are deactivated.

The second controlled valve (V2₁, V2₂, V2₃) and the third controlled valve (V3₁, V3₂, V3₃) are used to separate the branches (BR₁, BR₂, BR₃) from each other. Thus, when one of the branches (BR₁, BR₂, BR₃) is chosen, its corresponding second controlled valve (V2₁, V2₂, V2₃) and third controlled valve (V3₁, V3₂, V3₃) are open. For example, when the liquid standard sample or the unknown liquid sample of the second branch BR₂ is chosen, the second controlled valve V2₂ and the third controlled valve V3₂ of said second branch BR₂ are open whereas the other valves (V2₁, V2₃, V3₁, V3₃) are closed.

The fourth controlled valve V4 of the automated device comprises a first input I9 connected to the first output (O3₁, O3₂, O3₃) of the controlled valve system (VS₁, VS₂, VS₃). The first input I9 of the fourth valve V4 receives the vaporized standard sample during a calibration or the vaporized unknown sample during a measurement.

Moreover, the fourth controlled valve V4 includes a second input I10 that receives ambient air. Finally, the fourth controlled valve V4 comprises an output 09 connected to the first input I1 of the analyzer ANL. The output 09 of the fourth controlled valve V4 allows or blocks the passage of the vaporized standard sample, the ambient air or of the vaporized unknown sample to the analyzer ANL.

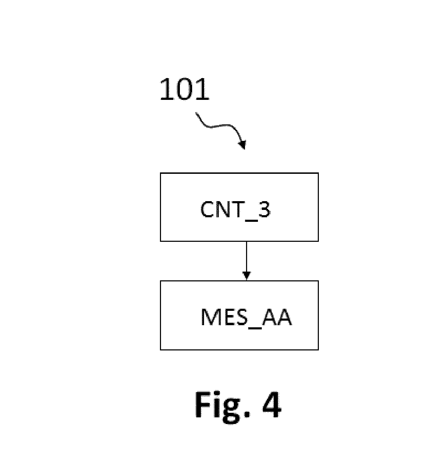

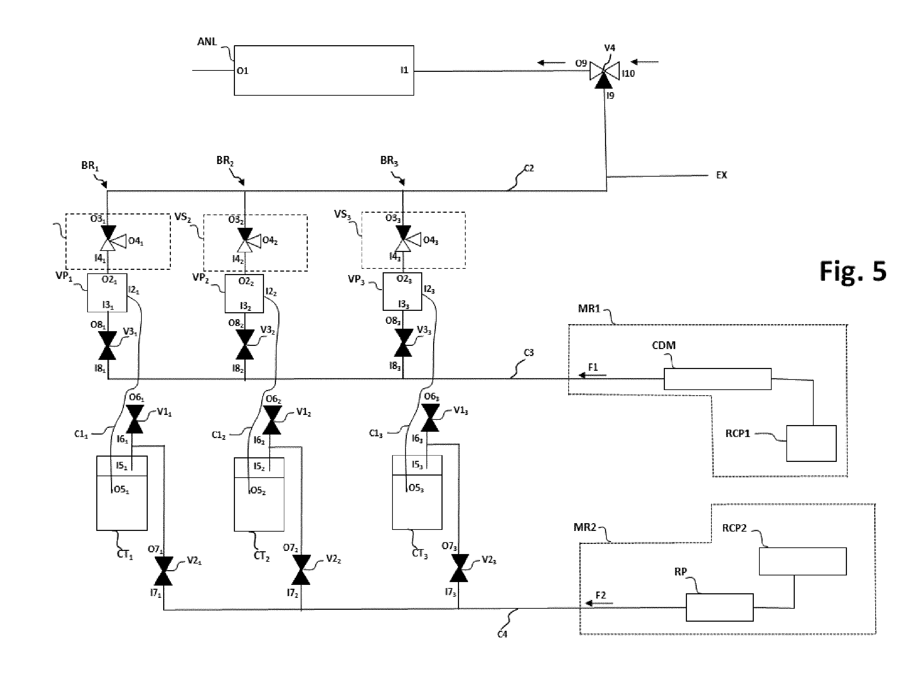

The figures 4 represents an example of a measurement step 101 of a measurement process of isotopic composition of an unknown liquid sample which is, in this case, an ambient air sample, said measurement process using the automated device of the figure 3. The figure 5 represents sub-steps of said measurement step 101.

The measurement step 101, as represented in the figure 4, comprises a connection CNT_3 of the second input I10 to the output 09 of the fourth controlled valve V4 as represented in the figure 5. The connection CNT_3 allows the passage of ambient air to the analyzer ANL. Subsequently, a measure MES_AA of isotopic composition of said ambient air sample, entered by the input I1 of the analyzer ANL, is realized by the analyzer ANL.

During the measurement step 101, the analyzer ANL is measuring ambient air.

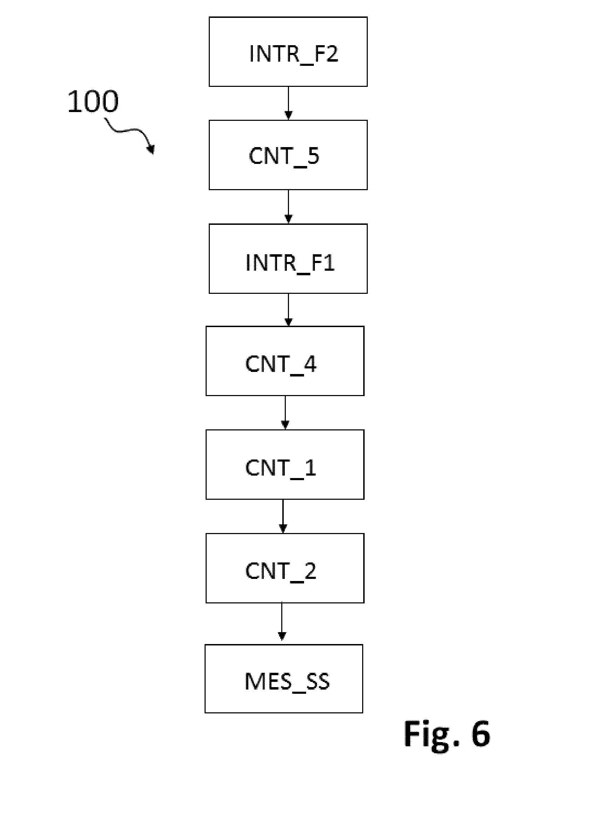

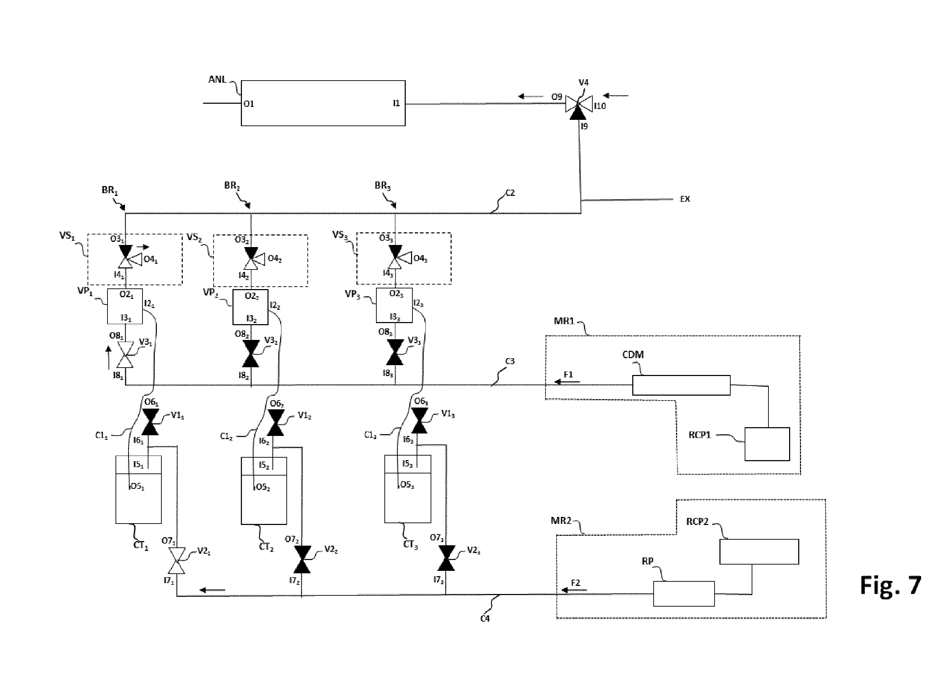

The figure 6 represents an example of a calibration step 100 of the measurement process. The figures 7 to 10 represent an example of sub-steps of said calibration step 100.

The calibration step 100, as represented in the figure 6, comprises an introduction INTR_F2 of the second gas flow F2 by the second regulation means MR2 in the fourth circuit C4.

Then, the calibration step 100 comprises a connection CNT_5 of the input I7₁ to the output O7₁ of the second controlled valve V2₁ of the first branch BR₁ as represented in the figure 7. The connection CNT_5 allows the passage of the second gas flow F2 from the fourth circuit C4 to the first container CT₁. The entry of the second gas flow F2 in the first container CT₁ increases the pressure in the headspace of the first container CT₁ until said pressure reaches a set point controlled by the pressure regulator RP of the second regulation means MR2. Thus, the increase of pressure in said headspace exerts a force on the surface of the liquid standard sample that is then pushed through the first circuit C1₁ of the first branch BR₁. The liquid standard sample is heated in the first vaporizer VP₁ that transforms said liquid standard sample into vapor named vaporized standard sample.

Subsequently, an introduction INTR_F1 of the first gas flow F1 in the third circuit C3 is realized by the first regulation means MR1.

Then, the calibration step 100 comprises a connection CNT_4 of the input I8₁ to the output O8₁ of the third controlled valve V3₁ of the first branch BR₁ as represented in the figure 7. The connection CNT_4 allows the passage of the first gas flow F1 from the third circuit C3 to the first vaporizer VP₁.

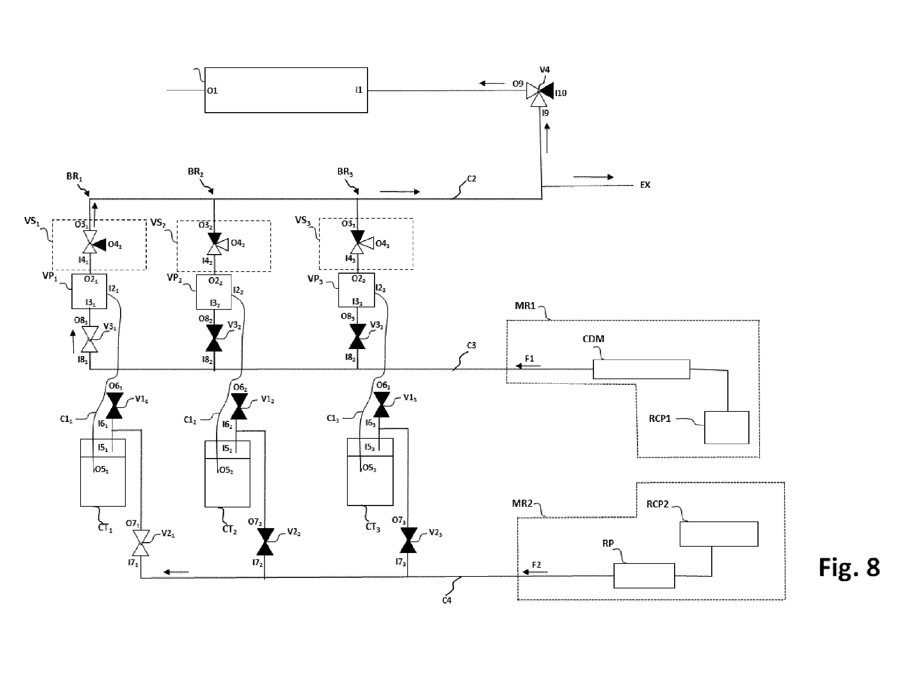

The entry of the first gas flow F1 in the first vaporizer VP₁ allows mixing of said first gas flow F1 with the vaporized standard sample contained in the first vaporizer VP₁. Furthermore, the mix of the first gas flow F1 with the vaporized standard permits to fix the rate of the flow mass of said mix. Indeed, the rate of the flow mass of the first gas flow F1 has to be larger than the flow needed at the input I1 of the analyzer ANL. The mix of vaporized standard sample with the first gas flow F1 is evacuated by the second output O4₁ of the controlled valve system VS₁ of the first branch BR₁ to reach a stable humidity flow. After the humidity flow reached, the connection between the input I4₁ and the second output O4₁ of the controlled valve system VS₁ of the first branch BR₁ is closed as represented in the figure 8.

Then, the calibration step 100 comprises a connection CNT_1 of the input I4₁ to the first output O3₁ of the controlled valve system VS₁ of the first branch BR₁ as represented in the figure 8. The connection CNT_1 allows the passage of the liquid standard sample from the first container CT₁, vaporized, by the first vaporizer VP₁, in the second circuit C2 of the third controlled valve V3₁ of the first branch BR₁.

Then, the calibration step 100 comprises a connection CNT_2 of the first input I9 to the output 09 of the fourth controlled valve V4 by controlling said fourth controlled valve V4. The connection CNT_2 allows the passage of the vaporized standard sample to the analyzer ANL. Most of the vaporized standard sample is flowing out through the exhaust EX.

Then, the calibration step 100 comprises a measure MES_SS of isotopic composition of the vaporized standard sample by the analyzer ANL. Indeed, the analyzer ANL aspires the flow of vaporized standard sample from the first output O3₁ of the controlled valve system VS₁ of the first branch BR₁. In one non-limiting embodiment, the duration of said measure MES_SS of isotopic composition of the vaporized standard sample is between 10 and 30 minutes depending on the accuracy and precision needed.

In a non-limiting embodiment, after the calibration step 100, the first output O3₁ and the second output O4₁ of the first controlled valve system VS₁ are closed whereas the output O6₁ of the first controlled valve V1₁ of the first branch BR₁ is opened. In this way, said output O6₁ of the first controlled valve V1₁ is connected to the ambient air which causes the air pressure inside the headspace of the first container CT₁ of the first branch BR₁ to be released. Moreover, the overpressure created in the first vaporizer VP₁ with the opening of the output O6₁ of the first controlled valve V1₁ pushes back the liquid standard sample into the first circuit C1₁ for a few seconds. Thus, since no more overpressure in the headspace of the first container CT₁ of the first branch BR₁ exists after this point, no liquid standard sample is pushed through the first circuit C1₁ of the first branch BR₁.

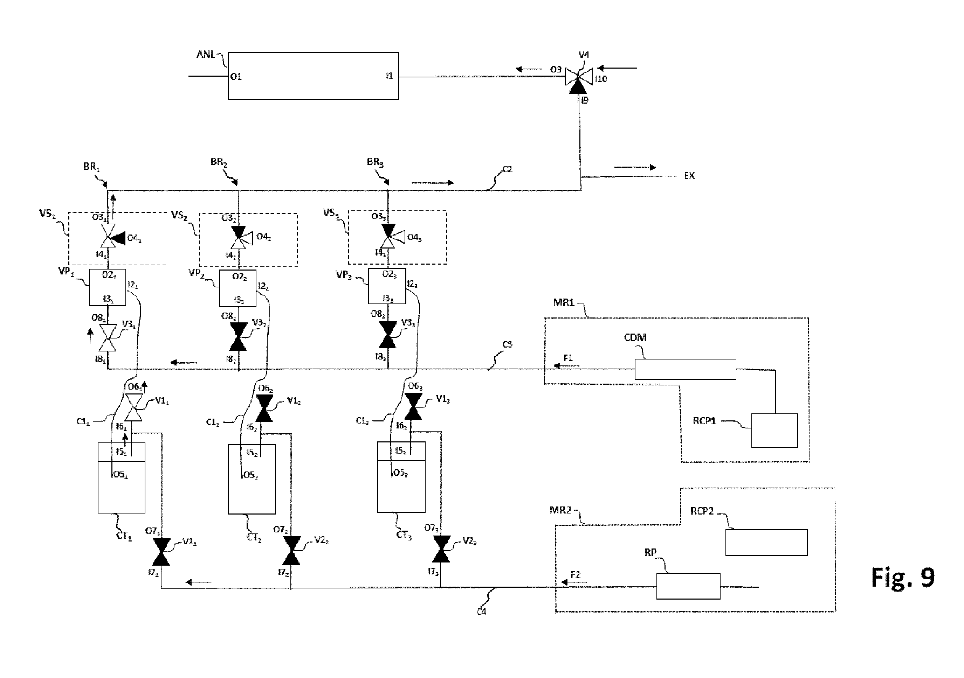

After the liquid standard sample have been pushed back in the first circuit C1₁, as represented in the figure 9, the first output O3₁ of the first controlled valve system VS₁ is opened for a few minutes to dry out the second circuit C2. Consequently, the first vaporizer VP₁ of the first branch BR₁ is dried out in order to prepare for the next liquid standard sample or for the next unknown liquid sample that will be measured. In the same way, the second circuit C2 is dried out by the second gas flow F2 that continues to cross into the first vaporizer VP₁ until the exhaust EX. In a non-limiting embodiment, the duration of the drying described above is on the order of a few minutes as the purpose is to release the pressure of the first container CT₁ of the first branch BR₁ and to stop the flow of the liquid standard sample in the first circuit C1₁ of the first branch BR₁.

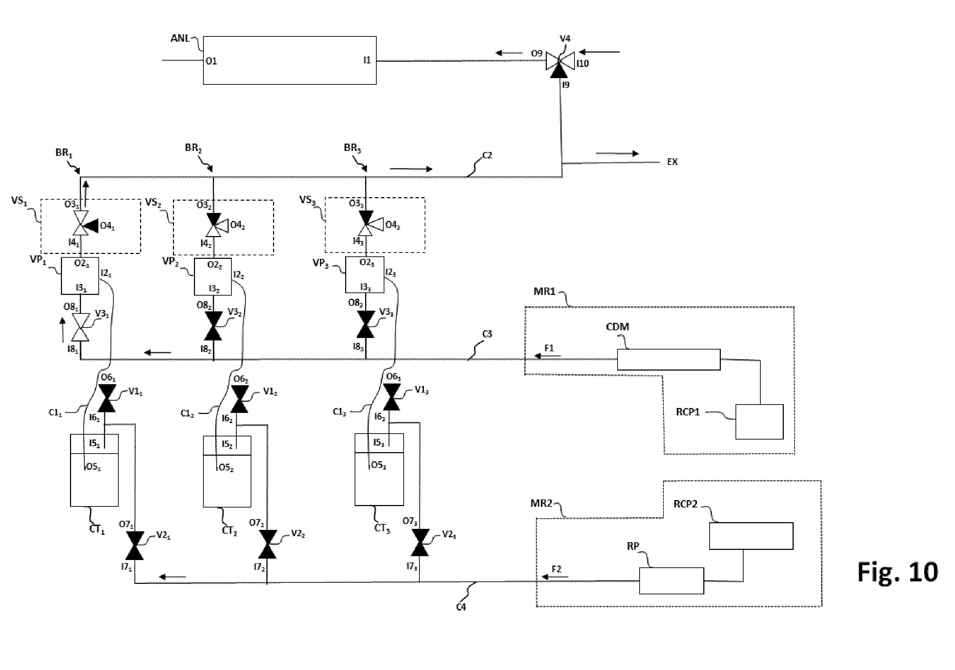

In a non-limiting embodiment of the invention, as represented in the figure 10, the output O6₁ of the first controlled valve V1₁ of the first branch BR₁ is closed since the pressure has been released from the headspace of the first container CT₁ of the first branch BR₁. The first gas flow F1 still flow through the first vaporizer VP₁ of the first branch BR₁ to dry out the second circuit C2. The duration of the drying described above is on the order of a few minutes in order to allow removal of any residual liquid standard sample or unknown liquid sample from the inside of the second circuit C2.

Advantageously, the present invention is able to run for more than 4 weeks in an autonomous mode.

Advantageously, the present invention can be connected to any water isotope spectral analyzer.

## Claims

1. An automated device adapted for the determination of the isotopic composition of an unknown liquid sample, comprising :
- at least a first branch (BR₁) comprising :
o a first vaporizer (VP₁) comprising :
▪ a first input (I2₁) constructed and arranged for receiving a liquid sample to be vaporized ;
▪ a second input (I3₁) constructed and arranged for receiving a first gas flow (F1) such as air flow ;
▪ an output (O2₁) constructed and arranged for outputting the vaporized liquid sample pushed out by said first gas flow (F1);
o a controlled valve system (VS₁) comprising an input (I4₁) connected to the output (O2₁) of the vaporizer (VP₁), a first output (O3₁) adapted to be connected to a gas analyzer and a second output (O4₁) left in the open air, said controlled valve system (VS₁) being constructed and arranged to allow or block the passage of the vaporized liquid sample to the gas analyzer and to reach a stable humidity of the vaporized liquid sample before being transported to the gas analyzer ;
o a first container (CT₁) constructed and arranged for containing said liquid sample comprising :
▪ an input (I5₁) constructed and arranged for receiving a second gas flow (F2) such as air flow ;
▪ an output (O5₁) constructed and arranged for transporting the liquid sample from the first container (CT₁);
o a first circuit (C1₁) connecting the output (O5₁) of the first container (CT₁) to the input (I2₁) of the first vaporizer (VP₁), said first circuit (C1₁) being constructed and arranged to bring the liquid sample to the first vaporizer (VP₁) ;
o a first controlled valve (V1₁) comprising an input (I6₁) connected to the input (I5₁) of the first container (CT₁) and an output (O6₁) left in the open air, said first controlled valve (V1₁) being constructed and arranged to allow or block the evacuating of the air contained in the first container (CT₁) by the output (O6₁) of the first controlled valve (V1₁) ;
- first regulation means (MR1) of the first gas flow (F1), said first regulation means (MR1) being connected to the second input (I3₁) of the first vaporizer (VP₁);
- second regulation means (MR2) of the second gas flow (F2), said second regulation means (MR2) being connected to the input (I5₁) of the first container (CT₁);
- a second circuit (C2) connected to the output (O3₁) of the controlled valve system (VS₁) ;
- a third circuit (C3) connecting the first regulation means (MR1) to the second input (I3₁) of the first vaporizer (VP₁) ;
- a fourth circuit (C4) connecting the second regulation means (MR2) to the input (I5₁) of the first container (CT₁).

2. An automated device according to claim 1, wherein the automated device comprises :
- a gas analyzer (ANL) comprising :
o an input (I1) constructed and arranged for receiving a gas to be analyzed ;
o an output (01) constructed and arranged for evacuating said gas after analysis.

3. An automated device, according to claim 2, wherein said automated device comprises an exhaust (EX) connected to the second circuit (C2), said exhaust (EX) being constructed and arranged to evacuate the flow surplus of vaporized liquid sample in the second circuit (C2) before its entry into the input (I1) of the gas analyzer (ANL).

4. An automated device, according to one of the previous claims, wherein the controlled valve system (VS₁) of the first branch (BR₁) is a three-way valve.

5. An automated device, according to claim 1 to 3, wherein the controlled valve system (VS₁) of the first branch (BR₁) is a combination of two two-way valves, said two two-way valves being constructed and arranged to be connected through a common point forming the input (I4₁) of the controlled valve system (VS₁).

6. An automated device, according to one of the previous claims, wherein the first regulation means (MR1) comprise:
- first means (RCP1) constructed and arranged for delivering dry gas ;
- a mass flow controller (CDM) connected to the first means (RCP1), said mass flow controller (CDM) being constructed and arranged to control the mass flow of the dry gas from the first means (RCP1), forming the first gas flow (F1).

7. An automated device, according to claim 6, wherein the first means (RCP1) constructed and arranged for delivering dry gas are either a first receptacle (RCP1) containing dry gas or a pump delivering gas connecting through a drying unit wherein said gas becomes dry.

8. An automated device, according to one of the previous claims, wherein the second regulation means (MR2) comprise :
- second means (RCP2) constructed and arranged for delivering compressed gas ;
- a pressure regulator (RP) connected to the second means (RCP2) constructed and arranged for delivering compressed gas, said pressure regulator (RP) being constructed and arranged to regulate the pressure of the compressed gas from said second means (RCP2) constructed and arranged for delivering compressed gas, forming the second gas flow (F2).

9. An automated device, according to claim 8, wherein the second means (RCP2) constructed and arranged for delivering compressed gas are either a second receptacle (RCP2) containing compressed gas or a pump delivering compressed gas.

10. An automated device, according to one of the previous claims, wherein said automated device comprises a plurality of branches (BR₁, BR₂, BR₃) mounted parallel, each branch (BR₁, BR₂, BR₃) being connected, at one end, to the second circuit (C2) and, at the other end, to the fourth circuit (C4) and comprising :
- a second controlled valve (V2₁, V2₂, V2₃) comprising an input (I7₁, I7₂, I7₃) and an output (O7₁, O7₂, O7₃) connected to the fourth circuit (C4), said second controlled valve (V2₁, V2₂, V2₃) being constructed and arranged to allow or block the passage of the second gas flow (F2) to a container (CT₁, CT₂, CT₃) ;
- a third controlled valve (V3₁, V3₂, V3₃) comprising an input (I8₁, I8₂, I8₃) connected to the third circuit (C3) and an output (O8₁, O8₂, O8₃), said third controlled valve (V3₁, V3₂, V3₃) being constructed and arranged to allow or block the passage of the first gas flow (F1) to an input (I3₁, I3₂, I3₃) of a vaporizer (VP₁, VP₂, VP₃).

11. An automated device, according to one of the previous claims, wherein the first circuit (C1₁, C1₂, C1₃) is a capillary tube.

12. An automated device, according to claim 11, wherein said automated device comprising the gas analyzer (ANL) comprises a fourth controlled valve (V4) comprising :
- a first input (I9) connected to a first output (O3₁, O3₂, O3₃) of a controlled valve system (VS₁, VS₂, VS₃), said first input (I9) being constructed and arranged for receiving the vaporized liquid sample ;
- a second input (I10) constructed and arranged for receiving ambient air;
- an output (09) connected to the first input (I1) of the gas analyzer (ANL), said output (09) being constructed and arranged to allow the passage of the vaporized liquid sample to the gas analyzer (ANL) or to allow the passage of ambient air from the second input (I10) to the gas analyzer (ANL).

13. Measurement process of isotopic composition of an unknown liquid sample, using the automated device according to the previous claim 12, comprising :
- a calibration step (100) comprising the following steps :
o introduction (INTR_F2) of the second gas flow (F2), by the second regulation means (MR2), in the fourth circuit (C4) ;
o introduction (INTR_F1) of the first gas flow (F1), by the first regulation means (MR1), in the third circuit (C3) ;
o connection (CNT_1) of one of the inputs (I4₁, I4₂, I4₃) to one of the first outputs (O3₁, O3₂, O3₃) of one of the controlled valve systems (VS₁, VS₂, VS₃) by controlling said controlled valve system (VS₁, VS₂, VS₃), said connection (CNT_1) allowing the passage of a liquid standard sample, from one of the containers (CT₁, CT₂, CT₃), vaporized, by one of the vaporizers (VP₁, VP₂, VP3), in the second circuit (C2) ;
o connection (CNT_2) of the first input (I9) to the output (09) of the fourth controlled valve (V4) by controlling said fourth controlled valve (V4), said connection (CNT_2) allowing the passage of the vaporized liquid standard sample to the analyzer (ANL) ;
o measurement (MES_SS) of isotopic composition of the vaporized liquid standard sample by the analyzer (ANL) ;
- a measurement step (101) comprising the following steps :
o connection (CNT_3) of the second input (110) to the output (09) of the fourth controlled valve (V4) by controlling said fourth controlled valve (V4), said connection (CNT_3) allowing the passage of ambient air to the analyzer (ANL) ;
o measurement (MES_AA) of isotopic composition in ambient air by the analyzer (ANL).

14. Measurement process, according to claim 13, wherein the calibration step (100) comprises the following steps :
o connection (CNT_4) of one of the inputs (I8₁, I8₂, I8₃) to one of the outputs (O8₁, O8₂, O8₃) of one of the third controlled valves (V3₁, V3₂, V3₃) said connection (CNT_4) allowing the passage of the first gas flow (F1) from the third circuit (C3) to one of the vaporizers (VP₁, VP₂, VP₃) ;
o connection (CNT_5) of one of the inputs (I7₁, I7₂, I7₃) to one of the outputs (O7₁, O7₂, O7₃) of one of the second controlled valves (V2₁, V2₂, V2₃), said connection (CNT_5) allowing the passage of the second gas flow (F2) from the fourth circuit (C4) to one of the containers (CT₁, CT₂, CT₃) and the passage of the liquid standard sample to one of the vaporizers (VP₁, VP₂, VP₃) through one of the first circuits (C1₁, C1₂, C1₃).
